## Europäisches Patentamt

## European Patent Office

## Office européen des brevets

(19)

(11) Veröffentlichungsnummer: **0 041 222**

**A1**

(12) # EUROPÄISCHE PATENTANMELDUNG

(21) Anmeldenummer: 81104052.6

(22) Anmeldetag: 27.05.81

(51) Int. Cl.³: **A 61 B 5/02**

(30) Priorität: 30.05.80 DE 3020567
04.10.80 DE 3037686

(43) Veröffentlichungstag der Anmeldung:
09.12.81 Patentblatt 81/49

(84) Benannte Vertragsstaaten:
AT BE CH FR GB IT LI LU NL SE

(71) Anmelder: Rieckmann, Jutta, Geb. Federlein
Hauptstrasse 12
D-6719 Altleiningen(DE)

(71) Anmelder: Rieckmann, Peter, Dr.
Hauptstrasse 12
D-6719 Altleiningen(DE)

(72) Erfinder: Rieckmann, Jutta, Geb. Federlein
Hauptstrasse 12
D-6719 Altleiningen(DE)

(72) Erfinder: Rieckmann, Peter, Dr.
Hauptstrasse 12
D-6719 Altleiningen(DE)

(74) Vertreter: Ratzel, Gerhard, Dr.
Seckenheimer Strasse 36a
D-6800 Mannheim 1(DE)

(54) **Verfahren und Vorrichtung zur unblutigen Messung des Blutdrucks.**

(57) Die Erfindung betrifft ein Verfahren und ein Vorrichtung zur sogenannten unblutigen Messung des Blutdrucks, bestehend aus einem flexiblen, aber nicht elastischen, mit Flüssigkeit gefüllten Beutel (1,13), einer variabel einstellbaren, aber starren, ringförmigen Klammer oder unelastischen Manschette (2), eine Druckpumpe (3,16), einem Vorratsbehälter (4) für Flüssigkeit, einer Anzeigevorrichtung (6) für Höchst- und Tiefstwerte des Druckes und einer verschliessbaren Rückleitung (7) für die Flüssigkeit aus dem Beutel (1,13) in den Vorratsbehälter (4). Der mit Flüssigkeit gefüllte Beutel (1) wird starr an einer Extremität angebracht und mit einem Druck beaufschlagt, der zwischen dem diastolischen und dem systolischen Druck liegt; die Höchst - und Tiefstwerte des Druckes der Flüssigkeit werden mit einem nahezu trägheitslosen Manometer (5) angezeigt. In der Phase des Druckaufbaus werden die Spitzenwerte (10) der Blutdruckpulse gemessen, wonach die Druckerhöhung beendet wird, wenn die Differenz (d) zweier zeitlich aufeinanderfolgender Spitzenwerte 0 ist oder unterhalb eines vorgegebenen Grenzwerts liegt. Ein den Druck in ein elektrisches Signal umsetzender Wandler (19) ist über eine Spannungs-Halteschaltung (20) mit einem Speicher (21) verbunden, dessen Eingangs-und Ausgangssignale einem Komparator (23) zugeführt werden. Wenn die Differenz der Signale 0 ist oder unterhalb des Grenzwertes liegt, wird die von der Pumpe (3,16) in den Beutel (1,13) führende Druckleitung (27) abgesperrt.

FIG. 1

## Verfahren und Vorrichtung zur unblutigen Messung des Blutdrucks

Gegenstand der vorliegenden Erfindung ist ein neues Verfahren und Vorrichtung zur sogenannten unblutigen Messung des Blutdrucks sowohl durch den Arzt als auch zur Selbstkontrolle durch den Patienten.

Der Blutdruck wird bisher unblutig nach dem Verfahren von Riva Rocci gemessen, bei dem eine Manschette aus Stoff und ein Druckbalg aus Gummi um den Oberarm gelegt werden. Der Druckbalg wird mittels Luft aufgepumpt, die langsam wieder abgelassen werden kann. Zunächst wird die Luft etwas über den systolischen Blutdruck aufgepumpt und so die Arterie abgequetscht. Mit Hilfe eines Ventils wird der Druck langsam abgelassen. Bei dem systolischen Druck treten Klopfgeräusche in der Arterie auf, die mit einem Stethoskop oder einem Mikrophon abgehört werden. Diese sogenannten Korotkoff-Geräusche verschwinden beim Erreichen des diastolischen Druckes wieder.

Dieses Verfahren und die Vielzahl der nach diesem Verfahren arbeitenden Vorrichtungen sind sehr ungenau und unterliegen erheblichen Schwankungen und Störungen selbst bei ein und demselben Patienten und ein und demselben Untersucher. Wirklich genaue Ergebnisse kann man bisher eigentlich nur bei der sogenannten blutigen Messung erhalten, was jedoch in der Praxis meist undurchführbar ist. Insbesondere für die Selbstüberwachung durch den Patienten sind eine Reihe von Geräten entwickelt worden, die das Abhören mit einem Mikrophon durchführen und die Ergebnisse elektrisch anzeigen. Die Ungenauigkeit und Störanfälligkeit dieser Geräte geht beispielsweise aus dem Bericht in der Zeitschrift "Test" der Stiftung Warentest vom Mai 1980 hervor.

Die vorliegende Erfindung hat sich die Aufgabe gestellt, das ungenaue und störanfällige Prinzip der unblutigen Blutdruckmessung nach Riva Rocci unter Ausnutzung der sogenannten Korotkoff-Geräusche durch eine bessere, zuverlässigere und dennoch unblutige Verfahrensweise zu ersetzen.

Diese Aufgabe wird dadurch gelöst, daß man einen flexiblen, aber nicht elastischen, mit einer Flüssigkeit gefüllten Beutel starr an einer Extremität anbringt, auf einen Druck bringt, der zwischen dem diastolischen und dem systolischen Druck liegt.und die Höchst- und Tiefstwerte des Druckes der Flüssigkeit mit einem nahezu trägheitslosen Manometer mißt. Die für Arzt und Patienten allein interessanten Höchst- und Tiefstwerte werden vorzugsweise mit Hilfe von Schleppzeigern oder einer entsprechenden elektronischen Anzeige ermittelt.

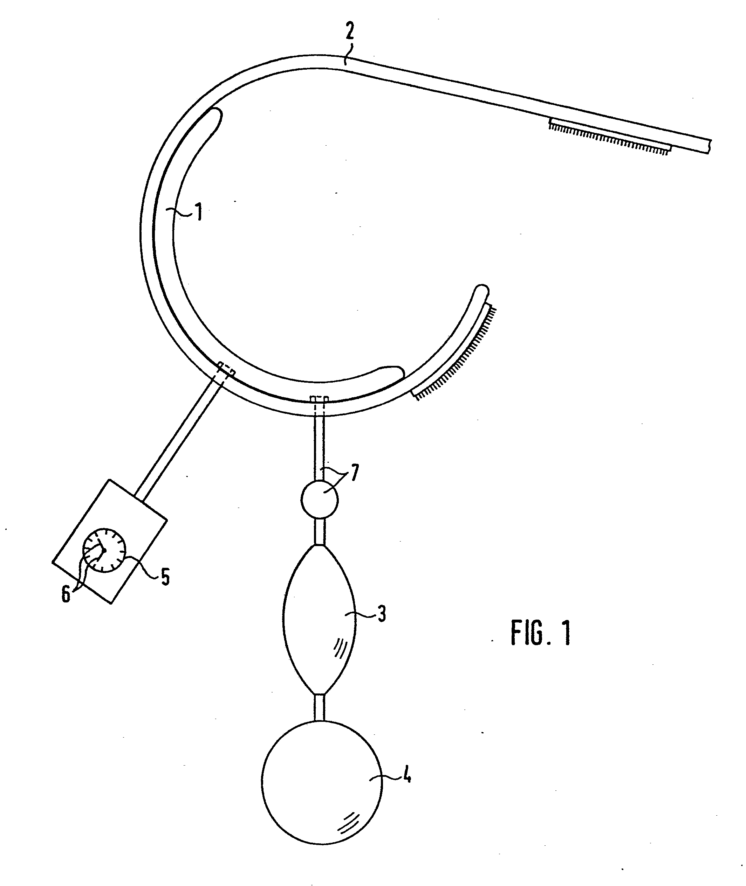

Die erfindungsgemäße Vorrichtung zur unblutigen Messung des Blutdrucks besteht aus einem flexiblen, aber nicht elastischen, mit Flüssigkeit gefülltem Beutel (1), einer variablen einstellbaren, aber starren ringförmigen Klammer oder unelastischen Manschetten (2), einer Druckpumpe (3), einem Vorratsbehälter für die Flüssigkeit (4), einem nahezu trägheitslosen Manometer (5), einer Anzeigevorrichtung für die Höchst- und Tiefstwerte des Druckes (6) und einer verschließbaren Rückleitung (7) für die Flüssigkeit aus dem Beutel in den Vorratsbehälter (Bezugszeichen siehe Figur 1).

Dadurch, daß der mit Flüssigkeit gefüllte Beutel flexibel, aber nicht elastisch ist, pflanzen sich die Druckschwankungen des Blutdrucksystems auf die Flüssigkeit fort. Sobald der Druck innerhalb des Beutels zwischen dem systolischen und diastolischen Druck liegt, werden auf die Flüssigkeit in dem Beutel Druckimpulse in Höhe des diastolischen und systolischen Drucks übertragen. Die Höchst- und Tiefstwerte werden mit einem nahezu trägheitslosen Manometer gemessen und beispielsweise durch Schleppzeiger oder elektronische Anzeigen ermittel und angezeigt.

Die starre Befestigung des Beutels an einer Extremität erfolgt mit einer variablen einstellbaren, aber starren, ringförmigen Klammer, welche den Beutel fest umschließt. Der Beutel ist einerseits mit einer Dosierpumpe und einem Vorratsbehälter für die Flüssigkeit versehen, um nach dem Schließen und Klammern den Innendruck im Beutel auf den Bereich zwischen systolischen und diastolischen Druck einstellen zu können. Andererseits ist eine verschließbare Rückleitung für die Flüssigkeit aus dem Beutel in den Vorratsbehälter vorgesehen, um nach der Messung die Flüssigkeit in den Vorratsbehälter zurückleiten zu können. Schließlich weist der Beutel ein nahezu trägheitsloses Manometer mit einer Anzeigevorrichtung für den Höchst- und Tiefstdruck auf, da die Druckbereiche dazwischen uninteressant sind. Der Beutel und die Schlauchleitungen müssen einerseits flexibel sein, um sich an die Extremität anschmiegen zu können. Sie dürfen jedoch andererseits nicht elastisch sein, weil dies zu Dämpfungen und Verfälschungen der Meßwerte führen würde. Geeignet sind daher flexible, aber nicht elastische Kunststoffolien oder gummierte Gewebe. Prinzipiell ließe sich der Druck auch durch Anziehen der Klammer oder der flexiblen, aber nicht elastischen Manschette einstellen, einfacher und zuverlässiger ist jedoch eine Vorrichtung, bei der man durch Nachpumpen von Flüssigkeit aus einem Vorratsbehälter den Druck einstellen kann und durch Öffnen der verschließbaren Rückleitung ihn zunächst nachregulieren und schließlich völlig entlasten kann.

Das neue Verfahren und die neue Vorrichtung geben die Druckverhältnisse in der Arterie exakt wieder, ohne daß es notwendig ist, die Arterie vollständig abzuquetschen. Weiterhin entfallen die Probleme, die sich aus dem Abhören der Korotkoff'schen Geräusche ergeben, sowie Fehler , die sich aus der verschiedenen Handhabung durch Arzt oder Patienten ergeben.

Die Messung kann erfindungsgemäß an einer beliebigen Extremität vorgenommen werden. Vorzugsweise können aber der Oberarm oder das Handgelenk als Meßpunkte gewählt werden. Prinzipiell können aber auch Oberschenkel oder Fußgelenk gewählt werden, insbesondere wenn man die Meßergebnisse über längere Zeit während verschiedener Tätigkeiten verfolgen will und die Meßergebnisse gegebenenfalls über einen kleinen Sender und Empfänger laufend verfolgen will.

In der nachfolgenden Figur 1 ist eine bevorzugte Ausführungsform der erfindungsgemäßen Vorrichtung näher erläutert. Hier bedeutet:

(1) den flexiblen, aber nicht elastischen, mit
Flüssigkeit gefüllten Beutel,

(2) eine variabel einstellbare, aber starre, ringförmige Klammer, oder flexible, aber unelastische
Manschette.

(3) eine Druckpumpe,

(4) einen Vorratsbehälter für die Flüssigkeit,

(5) ein nahezu trägheitslosen Manometer,

(6) eine Anzeige der Meßwerte und

(7) eine verschließbare Rückleitung für die Flüssigkeit aus dem Beutel in den Vorratsbehälter.

Nahezu trägheitslose Manometer sind an sich bekannt.
Bei Ihnen wird entweder die Streckung der Rohrfeder
oder die elastische Ausdehnung eines Blechgefäßes
nicht über Hebel und Zahnräder auf einen Zeiger übertragen, sondern durch Lichtreflektionen oder piezoelektrische Impulse in Meßwerte umgesetzt. Eine besonders einfache Ausführungsform besteht aus einer
Rohrfeder, einer gelochten runden Scheibe, die sich
zwischen einer Lichtquelle und einem Photoelement bewegt und bei der die Lichtimpulse gezählt werden.
Die Anzahl der Lichtimpulse wird digital angegeben,
so daß in besonders einfacher Weise eine Analog/
Digitalwandlung vorgenommen wird. Eine andere Ausführungsform weist am Druckaufnehmer einen Anker auf,
der die Induktion eines Differentialtransformators
verändert. Bei der piezo-elektrischen Messung wird
der Druck bzw. die Druckveränderung direkt in
Widerstandsänderungen oder Stromimpulse umgesetzt,
die in üblicher Weise digital angezeigt werden
können.

Nach einer weiteren bevorzugten Ausführungsform des
erfindungsgemäßen Verfahrens wird ferner die technische Aufgabe gelöst, den Druckaufbau im Beutel zu
beenden, sobald der für die Druchführung der Blutdruckmessung erforderliche statische Druck in der
richtigen Höhe erreicht ist. Da der Blutdruck des
Probanden vor der Messung noch unbekannt ist, war
es bisher nämlich in der Regel erforderlich, den
einzustellenden Druckbereich anderweitig zu ermitteln.

Zur Lösung dieser Aufgabe ist erfindungsgemäß vorgesehen, daß in der Phase des Druckaufbaus der Druck
in den Spitzenwerten der Blutdruckpulse gemessen wird,
und daß die Erhöhung beendet wird, wenn die Differenz
zweier zeitlich aufeinanderfolgender Spitzenwerte Null ist oder unterhalb eines vorgegebenen Grenzwertes liegt.

Die Erfindung geht von der Erkenntnis aus, daß bereits während des Druckaufbaus in dem Beutel Blutdruckpulse auf die Flüssigkeit übertragen werden. Diese Blutdruckpulse überlagern sich dem jeweils herrschenden statischen Druck und ihre Maxima sowie auch ihre Minima ändern sich in der Phase des Druckaufbaus. Wenn der statische Druck in dem Beutel einen zwischen dem diastolischen und dem systolischen Blutdruck liegenden Wert einnimmt, erzeugen die Blutdruckpulse in der Flüssigkeit gleichgroße maximale Druckwerte, die dem systolischen Blutdruck entsprechen und gleichgroße Druckminima, die dem diastolischen Blutdruck entsprechen. Nach dem erfindungsgemäßen Verfahren wird die Tatsache, daß von den Blutdruckpulsen gleiche Extremwerte in dem Beutel erzeugt werden, zur Erkennung der Tatsache benutzt, daß der statische Druck im Beutel nunmehr zwischen dem systolischen und dem diastolischen Blutdruck liegt. Wenn dieser Stand erreicht ist, wird die Druckerhöhung beendet und die Messung durchgeführt.

Eine Vorrichtung zur Durchführung dieser besonderen Ausführungsform des Verfahrens ist dadurch gekennzeichnet, daß ein den Druck in ein elektrisches Signal umsetzender Wandler über eine Spannungs-Halteschaltung mit einem Speicher verbunden ist, und
daß die Signale am Eingang und am Ausgang des
Speichers einem Komparator zugeführt werden, der
dann, wenn die Signaldifferenz Null ist oder unterhalb des Grenzwertes liegt, die von der Pumpe in
den Beutel führende Druckleitung absperrt.

Die Spannungs-Halteschaltung lädt sich jeweils auf
den Maximalwert auf und speichert somit die von den
Blutdruckpulsen erzeugten Druckmaxima. Jeder dieser
Maximalwerte wird in dem Komparator mit einem der
vorgehenden Maximalwerte verglichen. Der gewünschte
Druckzustand ist erreicht, wenn die aufeinanderfolgenden Maximalwerte annähernd gleich sind. In
gleicher Weise können für die Auswertung auch die
Minimalwerte der von den Blutdruckpulsen erzeugten
Druckschwankungen herangezogen werden, jedoch ist die
Auswertung der Maximalwerte in der Regel einfacher
zu realisieren.

In weiterer vorteilhafter Ausgestaltung der Erfindung
ist der Speicher von einem von den Blutdruckpulsen
abgeleiteten Impulssignal getaktet und bei jedem
Taktimpuls wird der an seinem Eingang anstehende
Spannungswert eingespeichert. Die Taktung des
Speichers wird also von den Blutdruckpulsen abgeleitet.

Dies ermöglicht einen exakten Vergleich der nacheinander auftretenden Sptzenwerte.

Als Pumpe für den Druckaufbau verwendet man zweckmäßigerweise eine Dosierpumpe. Besonders geeignet sind magnetangetriebene Membranpumpen.

In weiterer Ausgestaltung der Erfindung ist ein Ventil vorgesehen, das in einer ersten Schaltstellung die Pumpe mit dem Beutel verbindet und in einer zweiten Schaltstellung die zu dem Beutel führende Druckleitung absperrt. Die Absperrung der Druckleitung erfolgt dann, wenn der Komparator angesprochen hat.

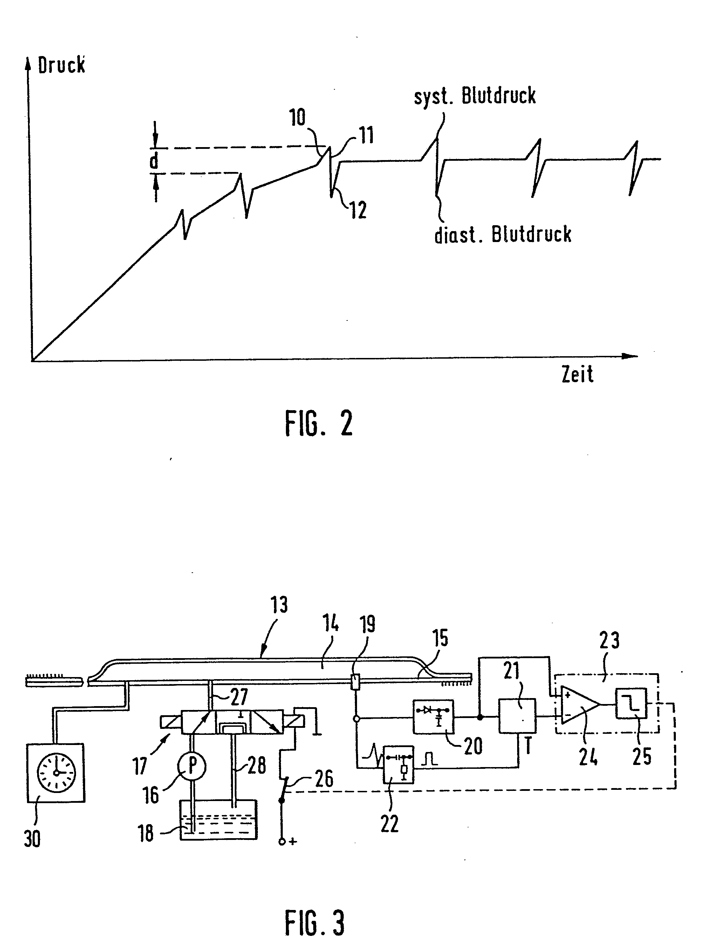

Im folgenden wird unter Bezugnahme auf die Figuren 2 und 3 ein bevorzugtes Ausführungsbeispiel der Erfindung näher erläutert.

Es zeigen:

Figur 2 ein schematisches Diagram des zeitlichen Druckverlaufs bei der Einstellung des für die Blutdruckmessung geeigneten statischen Druckes und

Figur 3 eine schematische Darstellung der Meß- und Steuerungseinrichtung zur Einstellung des richtigen statischen Druckes im Beutel.


Die in Fig. 2 dargestellte Druckkurve zeigt den zeitlichen Verlauf des Druckaufbaus in dem Beutel. Der Druck steigt zunächst langsam, beim Erreichen der Füllungsgrenze aber schneller. In diesem Bereich werden die Blutdruckimpulse auf die Flüssigkeit übertragen. Dabei entsteht bei jedem Blutdruckpuls eine positive Spannungsspitze 10, die oberhalb des statischen Druckes liegt, eine steile negative Flanke 11 und schließlich ein kurzzeitiges Druckminimum 12. Der zeitliche Abstand dieser Blutdruckpulse entspricht der Pulsfrequenz. In demjenigen Druckbereich, der für die Blutdruckmessung geeignet ist, entspricht der Druckspitzenwert, der bei jedem Blutdruckpuls entsteht, dem systolischen Blutdruck und der Minimalwert entspricht dem diastolischen Blutdruck. Die Erkennung, ob dieser Druckbereich erreicht ist, erfolgt durch Vergleichen der Sptzenwerte der Drücke an zwei aufeinanderfolgenden Blutdruckpulsen. Wenn diese Spitzenwerte eine Differenz d haben, die oberhalb des vorgegebenen Grenzwertes liegt, dann muß der statische Druck noch weiter erhöht werden, weil die positiven und negativen Spitzenwerte noch nicht dem systolischen bzw. diastolischen Blutdruck entsprechen. Wenn die Differenz d der Spitzenwerte hinreichend klein geworden ist, ist ein für die Messung geeigneter statischer Druck in dem Beutel aufgebaut, so daß der weitere Druckaufbau beendet und der Beutel nunmehr abgesperrt wird. In diesem Zustand erfolgt die Messung der Blutdruckwerte.

Die hierfür geeignete Meßeinrichtung ist in Fig. 3 schematisch dargestellt. Der Beutel 13, der die mit Flüssigkeit gefüllte Kammer 14 umschließt, ist an einem Manschettenband 15 befestigt,daß z.B. um den Arm des Probanden gewickelt werden kann. Zum Aufpumpen der Kammer 14 dient die Dosierpumpe 16, die über das Magnetventil 17 Flüssigkeit aus dem Tank 18 in die Kammer 14 fördert. Der Druck in der Kammer 14 wird durch einen Drucksensor 19 in eine elektrische Spannung umgewandelt. Der Ausgang des Drucksensors 19 ist an eine Spannungs-Halteschaltung 20 angeschlossen, die einen Speicherkondensator enthält, welcher über eine Diode aufgeladen wird. Die Spannungshalteschaltung 20 lädt sich bei jeder positiven Spannungsspitze des Druckverlaufs (Fig. 2) auf den jeweiligen Sptzenwert auf und hält diesen Wert auch dann, wenn die Spannung anschließend wieder abfällt. Der Ausgang der Spannungs-Halteschaltung 20 ist mit dem Eingang eines getakteten Speichers 21 verbunden.

Der Takt T, mit dem der Speicher 21 getaktet ist, wird über eine Differenzierschaltung 22 von den Signalen des Wandlers 19 abgeleitet. Die Differenzierschaltung 22, die z.B. ein RC-Glied enthält, erzeugt bei jedem Blutdruckpuls einen Taktimpuls, der an den Takteingang T des Speichers 21 gelegt wird. Bei Auftreten eines Taktimpulses übernimmt der Speicher 21 das an seinem Eingang anstehende Ausgangssignal der Spannungs-Halteschaltung 20. Der Speicher 21 speichert daher die Spitzenspannung des jeweils letzten Blutdruckpulses.

Die Ausgangssignale der Spannungs-Halteschaltung 20 und des Speichers 21 werden einem Komparator 23 zugeführt, der ein Ausgangssignal erzeugt, wenn die Differenz d der beiden Spannungen einen vorgegebenen Grenzwert unterschreitet. Der Komparator 23 erhält einen Differenzverstärker 24 und eine Schwellwertschaltung 25, an der die Größe des Grenzwertes eingestellt werden kann. Das Ausgangssignal des Komparators 23 steuert einen Schalter 26, der das Magnetventil 17 in den zweiten Schaltzustand versetzt, in dem es die in die Kammer 14 hineinführende Druckleitung 27 abgesperrt, wenn der Komparator 23 ein Ausgangssignal liefert. In dieser zweiten Schaltstellung (Mittelstellung) des Ventils 17, wird der Ausgang der Pumpe 16 mit einer in den Tank 18 hineinführenden Rücklaufleitung 28 verbunden, während die Leitung 27 abgesperrt wird. Wenn dieser Zustand erreicht ist, bleibt der statische Druck in der Kammer 14 konstant. Die Maximal- und Minimalwerte der Blutdruckpulse, die dem systolischen bzw. diastolischen Blutdruck entsprechen, können nun an dem Druckmeßgerät 30, das für jeden der beiden Druckwerte einen Zeiger aufweist, abgelesen werden. Anschließend wird das Ventil 17 in seine dritte Schaltstellung gebracht, in der es die Leitung 27 mit der Rücklaufleitung 28 verbindet, um die Druckflüssigkeiten in den Tank 18 hinein zu entleeren.

- 1 -

A n s p r ü c h e
────────────────

1. Verfahren zur unblutigen Messung des Blutdrucks, dadurch gekennzeichnet, daß man einen flexiblen, aber nicht elastischen, mit einer Flüssigkeit gefüllten Beutel starr an eine Extremität anbringt, auf einen Druck bringt, der zwischen dem diastolischen und systolischen Druck liegt und die Höchst- und Tiefstwerte des Druckes der Flüssigkeit mit einem weitgehend trägheitslosen Manometer mißt.

2. Verfahren gemäß Anspruch 1, dadurch gekennzeichnet, daß man die Höchst- und Tiefstwerte des Drucks mittels trägheitslosen Manometern mit Schleppzeigern oder mit elektronischer Anzeige ermittelt.

3. Verfahren nach Anspruch 1 oder 2, dadurch gekennzeichnet, daß in der Phase des Druckaufbaus der Druck in den Spitzenwerten der Blutdruckpulse gemessen wird, und daß die Druckerhöhung beendet wird, wenn die Differenz zweier zeitlich aufeinanderfolgender Spitzenwerte 0 ist oder unterhalb eines vorgegebenen Grenzwertes liegt.

4. Vorrichtung zur unblutigen Messung des Blutdrucks, bestehend aus einem flexiblen, aber nicht elastischen, mit Flüssigkeit gefüllten Beutel (1), einer variablen einstellbaren, aber starren, ringförmigen Klammer oder unelastischen Manschette (2), einer Druckpumpe (3), einem Vorratsbehälter für Flüssigkeit (4), einem nahezu trägheitslosen Manometer (5), einer Anzeigevorrichtung für die Höchst- und Tiefstwerte des Druckes (6) und einer verschließbaren Rückleitung (7) für die Flüssigkeit aus dem Beutel in den Vorratsbehälter.

5. Vorrichtung gemäß Anspruch 4,
dadurch gekennzeichnet,
daß die Anzeigevorrichtung (6) aus Schleppzeigern oder einer elektronischen Anzeige besteht.

6. Vorrichtung gemäß Ansprüchen 4 oder 5,
dadurch gekennzeichnet,
daß die variabel einstellbare, aber starre ringförmige Klammer (2) um den Oberarm oder das Handgelenk paßt.

7. Vorrichtung zur Durchführung des Verfahrens nach Anspruch 3, dadurch gekennzeichnet, daß ein den Druck in ein elektrisches Signal umsetzender Wandler (19) über eine Spannungs-Halteschaltung (20) mit einem Speicher (21) verbunden ist, und daß die Signale am Eingang und am Ausgang des Speichers (21) einem Komparator (23) zugeführt werden, der dann, wenn die Signalfrequenz Null ist oder unterhalb des Grenzwertes liegt, die von der Pumpe (16) in den Beutel (13) führende Druckleitung (27) absperrt.

8. Vorrichtung nach Anspruch 7,
dadurch gekennzeichnet,
daß der Speicher (21) von einem von den Blutdruckpulsen abgeleiteten Impulssignal getaktet ist und bei jedem Taktimpuls den an seinem Eingang anstehenden Spannungswert einspeichert.

9. Vorrichtung nach Anspruch 7 oder 8,
dadurch gekennzeichnet,
daß ein Ventil (17) vorgesehen ist, das in einer ersten Schaltstellung die Pumpe (16) mit dem Beutel (13) verbindet und in einer zweiten Schaltstellung die zu dem Beutel (13) führende Druckleitung (27) absperrt.

10. Vorrichtung nach Anspruch 9, dadurch gekennzeichnet, daß das Ventil (17) eine dritte Schaltstellung aufweist, in der es die zu dem Beutel (13) führende Druckleitung (27) mit einem Tank (18) verbindet.


11. Vorrichtung nach Anspruch 9, dadurch gekennzeichnet, daß das Ventil (17) von dem Ausgangssignal des Komparators (23) gesteuert ist.

FIG. 1

FIG. 2

FIG. 3

**0041222**

Europäisches
Patentamt

**EUROPÄISCHER RECHERCHENBERICHT**

Nummer der Anmeldung

EP 81 10 4052

| Kategorie | Kennzeichnung des Dokuments mit Angabe, soweit erforderlich, der maßgeblichen Teile | betrifft Anspruch | KLASSIFIKATION DER ANMELDUNG (Int. Cl.3) |
|---|---|---|---|
| | **EINSCHLÄGIGE DOKUMENTE** | | |
| | FR - A - 2 352 530 (BARR & STROUD Ltd.) | 1,4,6 | A 61 B 5/02 |
| | x Seite 4, Zeilen 3-31; Seite 6, Zeilen 3-21; Seite 8, Zeile 13 - Seite 9, Zeile 1 und Abbildungen 2,3,6 x | | |
| | -- | | |
| | DE - A - 1 817 089 (A.B. BOFORS) | 1 | |
| | x Seite 4, Zeile 3 - Seite 5, Zeile 8; Seite 6, Zeile 23 - Seite 7, Zeile 7; Seite 8, Zeilen 1-12 und Abbildungen 1,2 x | | |
| | -- | | **RECHERCHIERTE SACHGEBIETE (Int. Cl.3)** |
| | FR - A - 2 405 694 (A.H. SAKS) | 1,4 | A 61 B 5/02 |
| | x Seite 6, Zeile 39 - Seite 8, Zeile 3; Seite 10, Zeile 15 - Seite 11, Zeile 40; Abbildungen 1-10 x | | |
| | -- | | |
| | US - A - 3 901 217 (T.W. CLARK) | 2,5 | |
| | x Zusammenfassung; Spalte 3, Zeilen 53-67; Spalte 5, Zeilen 17-37; Spalte 6, Zeilen 22-48; Spalte 8, Zeile 62 - Spalte 10; Zeile 9 und Abbildungen 7-9, 16-18 x | | |
| | -- | | **KATEGORIE DER GENANNTEN DOKUMENTE** |
| | FR - E - 42 276 (J.R. CARRE) | 1,4,6 | X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | x Seite 1, Zeile 1 - Seite 2, Zeile 60 und Abbildungen 1 + 2 x | | |
| | -- | | |
| | ./. | | |
| | | | &: Mitglied der gleichen Patentfamilie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 2-09-1981 | RIEB |

EPA form 1503.1   06.78

| | EINSCHLÄGIGE DOKUMENTE | | KLASSIFIKATION DER ANMELDUNG (Int.Cl. ³) |
|---|---|---|---|
| Kategorie | Kennzeichnung des Dokuments mit Angabe, soweit erforderlich, der maßgeblichen Teile | betrifft Anspruch | |
| | GB - A - 2 023 849 (MATSUSHITA ELECTRIC WORKS, Ltd.) | 1,7-9, 11 | |
| | ✶ Zusammenfassung; Seite 2, Zeilen 59-115; Seite 3, Zeilen 48-107; Seite 4, Zeilen 65-104; Seite 5, Zeilen 38-103 und Abbildung 2 ✶ | | |
| P | & WO - A - 79/00294 EP - A - 0 014 720 | | |
| | --- | | RECHERCHIERTE SACHGEBIETE (Int. Cl. ³) |
| | US - A - 4 058 117 (W.J. KASPARI et al/PALO ALTO RESEARCH ASSOCIATES) | 1-6 | |
| | ✶ Zusammenfassung; Spalte 3, Zeilen 14-44; Spalte 5, Zeilen 32-51; Spalte 6, Zeile 66 – Spalte 7, Zeile 44 und Abbildungen 1-6 ✶ | | |
| | --- | | |
| | ELEKTRONIK, Band 20, Heft 12, Dezember 1971 MÜNCHEN (DE) D. BENDA "Elektronische Patienten- überwachung", Teil 3, Seiten 427-432 | 2,3,5 | |
| | ✶ Seite 427, Abschnitt "3.4.2. Funktion und Aufbau eines S-D-Blutdruckmonitors" ✶ | | |
| | --- | | |
| | US - A - 4 009 709 (W.T. LINK et al./AMERICAN OPTICAL CORP.) | 1,3 | |
| | ✶ Zusammenfassung; Spalte 2, Zeilen 26-46; Spalte 3, Zeile 52 – Spalte 4, Zeile 55; Spalte 6, Zeilen 21-39; Abbildungen 1-5 ✶ | | |
| | -- ./. | | |

**Europäisches Patentamt**

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P | US - A - 4 261 368 (D. DANNA et al./WELCH ALLYN INC.)<br><br>* Zusammenfassung; Spalte 2, Zeile 65 - Spalte 3, Zeile 12; Spalte 4, Zeile 27 - Spalte 5, Zeile 23; Abbildungen 1-3 * | 1,8 |

KLASSIFIKATION DER ANMELDUNG (Int.Cl. 3)

RECHERCHIERTE SACHGEBIETE (Int. Cl. 3)

EPA Form 1503.2 06.78